# FASCICULE DE BREVET EUROPEEN

(11) **EP 4 188 148 B1**
(45) Date de publication et mention de la délivrance du brevet: **18.02.2026**
(21) Numéro de dépôt: 21749677.7
(22) Date de dépôt: 12.07.2021
(51) Int. Cl.: A44B 18/00, B29C 41/28, B29C 43/52, B29C 43/50, B29C 43/46, A61F 13/62

(54) **DISPOSITIF DE RETENUE, ARTICLE ABSORBANT COMPRENANT UN TEL DISPOSITIF, ET PROCEDE DE FABRICATION D'UN TEL DISPOSITIF**
HALTEVORRICHTUNG, SAUGFÄHIGER ARTIKEL MIT SOLCH EINER VORRICHTUNG UND VERFAHREN ZUR HERSTELLUNG SOLCH EINER VORRICHTUNG
RETAINING DEVICE, ABSORBENT ITEM COMPRISING SUCH A DEVICE, AND METHOD FOR MANUFACTURING SUCH A DEVICE

(30) Priorité: 03.08.2020 FR 2008241
(43) Date de publication de la demande: 07.06.2023
(73) Titulaire: Aplix, 44850 Le Cellier (FR)
(72) Inventeur: PICOT, Lionel, 44850 LE CELLIER (FR)
(74) Mandataire: Cabinet Beau de Loménie
(86) Numéro de dépôt international: PCT/FR2021/051295
(87) Numéro de publication internationale: WO 2022/029378

(56) Documents cités:
- EP-A1- 1 702 599
- FR-A1- 3 050 624
- US-A- 3 312 583
- US-A- 5 884 374
- US-A1- 2005 060 849
- US-B1- 6 206 679

## Description

### Domaine Technique

Le présent exposé concerne le domaine des systèmes de retenue, en particulier des systèmes de fermeture ou d'anti-glissement. L'exposé concerne plus particulièrement les systèmes de retenue à éléments de retenue, en particulier les systèmes de retenue auto-agrippantes, dont les éléments de retenue peuvent être retenus dans des boucles ou analogues.

L'exposé concerne également le domaine des procédés et appareillages de fabrication associés.

L'exposé concerne encore le domaine des articles absorbants du type couche-culotte pour bébé ou couche d'incontinence pour adulte comprenant un système de retenue auto-agrippante de la couche.

### Technique antérieure

Les systèmes de retenue comprenant des éléments de retenue portés par une base sont bien connus et utilisés dans de nombreux domaines d'application, conduisant de fait à la réalisation de multiples formes d'éléments de retenue, en particulier des crochets, destinés à coopérer mutuellement ou avec des éléments complémentaires tels que des boucles.

Une problématique récurrente vis-à-vis de tels produits concerne l'effort de retenue appliqué, compte tenu notamment des dimensions fortement réduites des éléments de retenue et des contraintes de fabrication associés.

En effet, la réalisation de crochets présentant des résistances élevées conduit naturellement à un surdimensionnement, ce qui nuit à la douceur du système et donc au ressenti par l'utilisateur, ce qui est fortement pénalisant dans certains domaines notamment dans le domaine de l'hygiène, et conduit également à une consommation accrue de matière ce qui est pénalisant en termes de coût, et également pénalisant en termes de fabrication dans la mesure où la réalisation de pièces volumineuses conduit à des durées de refroidissement de la matière accrues, augmentant de fait le temps d'occupation des lignes de production.

Le document WO2017187097 divulgue un dispositif de retenue comprenant des éléments de retenue portés par une base et présentant des caractéristiques dimensionnelles et géométriques particulières. Les documents US5884374, US2005/060849 et FR3050624 présentent d'autres exemples de dispositifs de retenue connus.

Le présent exposé vise ainsi à répondre à ces différentes problématiques en améliorant encore les dispositifs connus.

### Présentation de l'exposé

Selon un premier aspect, l'exposé concerne un dispositif de retenue tel que défini dans la revendication 1, comprenant
- une base s'étendant selon une direction longitudinale présentant une face supérieure et une face inférieure,
- une pluralité d'éléments de retenue s'étendant de la face supérieure de la base, chaque élément de retenue présentant une valeur selon une première dimension géométrique, ladite valeur selon une première dimension géométrique étant l'une parmi :
- la hauteur des éléments de retenue, mesurée, pour chaque élément de retenue, entre une extrémité inférieure de l'élément de retenue reliée à la base et une extrémité supérieure de l'élément de retenue opposée à l'extrémité inférieure,
- une dimension mesurée selon une vue en coupe perpendiculaire à la base, en particulier une vue en coupe perpendiculaire à la base et parallèle à une direction transversale,
- une dimension mesurée selon une vue parallèle à la base et à distance des éléments de retenue,
- une dimension transversale des éléments de retenue, mesurée parallèlement à la face supérieure de la base,
- une dimension diamétrale d'une portion des éléments de retenue dans le cas où les éléments de retenue présentent, au moins dans certaines portions, une symétrie de révolution,
- l'épaisseur d'une tige des éléments de retenue ou la largeur de têtes des éléments de retenue, mesurée dans un plan parallèle à la face supérieure de la base,
le dispositif de retenue présentant au moins deux zones de retenue et une zone intermédiaire disposée entre les deux zones de retenue et reliant les deux zones de

retenue, les éléments de retenue disposés dans les première et deuxième zones de retenue ayant des valeurs selon la première dimension géométrique qui sont supérieures aux valeurs selon la première dimension géométrique des éléments de retenue disposés dans la zone intermédiaire, l'ensemble formé par la base et les éléments de retenue présentant un grammage compris entre 10 et 120 g/m². Optionnellement, les zones de retenue et la zone intermédiaire forment des zones allongées en forme de ruban dans un ruban commun, les zones allongées et le ruban commun étant optionnellement allongés selon la direction longitudinale. Optionnellement, la zone intermédiaire présente au moins une partie dans laquelle la base présente une épaisseur inférieure à une épaisseur de la base dans les zones de retenue.

Optionnellement dans la zone intermédiaire la base présente une ligne de soudure.

Optionnellement, dans la zone intermédiaire, la base présente des vides.

Optionnellement dans la zone intermédiaire, la base possède un état de la matière différent de l'état de matière qu'elle présente dans les zones de retenues.

Optionnellement, dans les zones de retenue, les éléments de retenue présentent une valeur selon la première dimension géométrique qui est sensiblement constante.

Optionnellement, considérée dans le sens allant de la première zone de retenue à la deuxième zone de retenue, la zone intermédiaire présente successivement des éléments de retenue dont la valeur selon la première dimension géométrique décroît puis des éléments de retenue dont la valeur selon la première dimension géométrique croît.

Optionnellement, les valeurs selon la première dimension géométrique sont mesurées selon une vue en coupe perpendiculaire à la base, en particulier une vue en coupe perpendiculaire à la base et parallèle à la direction transversale.

Optionnellement, les valeurs selon la première dimension géométrique sont mesurées selon une vue parallèle à la base et à distance des éléments de retenue.

Optionnellement, toute ligne droite passant par au moins un élément de retenue et s'étendant selon la direction allant de la première zone de retenue à la deuxième zone de retenue en passant par lesdites première et deuxième zones de retenue, croise au moins 3 éléments de retenue, en particulier au moins 5 éléments de retenue dans chacune des première et deuxième zones de retenue. Optionnellement, toute ligne droite passant par au moins un élément de retenue et s'étendant selon la direction allant de la première zone de retenue à la deuxième zone de retenue en passant par lesdites première et deuxième zones de retenue, croise au moins 1 élément de retenue, en particulier au moins 2 éléments de retenue dans la zone intermédiaire. On peut prévoir que toute droite du type défini ci-dessus croise jusqu'à 70 éléments de retenue, voire 100 éléments de retenue dans chacune des première et deuxième zones de retenue et, optionnellement, jusqu'à 10 éléments de retenue, voire jusqu'à 30 éléments de retenue dans la zone intermédiaire.

Optionnellement, au moins dans les zones de retenue, les éléments de retenue sont chacun formés d'une tige surmontée d'une tête dépassant de la tige, une partie au moins des éléments de retenue de la zone intermédiaire étant optionnellement dépourvus de tête.

Optionnellement, au moins dans les zones de retenue, les éléments de retenue sont répartis suivant un motif régulier et répétitif.

Optionnellement les éléments de retenue sont régulièrement répartis, par exemple sous forme de lignes ou de colonne ou encore en quinconce.

Optionnellement, le dispositif comprend en outre un substrat portant la base, le substrat comprenant optionnellement une couche de matériau non-tissé.

Optionnellement, l'ensemble formé par la base et les éléments de retenue présente un grammage compris entre 30 et 80 g/m² (grammes par mètre carré), dans certains cas entre 30 et 70 g/m², plus particulièrement entre 50 et 70 g/m². Lorsque le dispositif comprend un substrat, ces valeurs de grammage sont en particulier vérifiées pour l'ensemble comprenant la base et les éléments de retenue, sans le substrat.

En particulier, la base et les éléments de retenue sont présents dans les zones de retenue et dans la zone intermédiaire.

En particulier, la zone intermédiaire relie les deux zones de retenue de manière jointive, sans espace entre la zone intermédiaire et, respectivement, chacune des deux zones de retenue.

En particulier, la première dimension géométrique est une dimension selon une direction donnée. Ainsi qu'il a été indiqué, il s'agit par exemple de la hauteur des éléments de retenue. Il peut cependant s'agir d'une autre dimension que la hauteur, par exemple une dimension transversale des éléments de retenue, mesurée parallèlement au plan de la base. Lorsque les éléments de retenue présentent, au moins dans certaines portions, une symétrie de révolution, la première dimension géométrique peut être une dimension diamétrale de ces portions. Bien entendu, ces dimensions sont combinables. Ainsi, en plus de la première dimension géométrique, les éléments de retenue peuvent présenter une deuxième dimension géométrique pour laquelle il est vérifié que les éléments de retenue disposés dans les première et deuxième zones de retenue ont des valeurs selon la deuxième dimension géométrique qui sont supérieures aux valeurs selon la deuxième dimension géométrique des éléments de retenue disposés dans la zone intermédiaire.

Dans la zone intermédiaire, les éléments de retenue présentent des caractéristiques géométriques qui sont dégradées par rapport aux mêmes caractéristiques des éléments de retenue des zones de retenue de sorte que, au moins pour la première dimension géométrique, les éléments de retenue de la zone intermédiaire présentent des valeurs moindres que ceux des zones de retenue.

Cependant, dans une certaine mesure, les éléments de retenue de la zone intermédiaire peuvent contribuer à l'effort de retenue. Ils permettent de disposer d'éléments de retenue pleinement efficaces sur les larges zones que constituent les zones de retenue, sans interruption entre ces zones pleinement efficaces alors que, du fait de leurs valeurs réduites pour la première dimension géométrique, les éléments de retenue de la zone de retenue représentent des masses moindres que celles des éléments de retenue des zones de retenue, ce qui permet de réaliser un dispositif de retenue efficace, de faible grammage.

Le dispositif de retenue présente une flexibilité accrue dans la ou les zones intermédiaires par rapport aux zones de retenue. A nombre égal d'éléments de retenue, le dispositif est donc non seulement plus léger mais également plus flexible qu'un dispositif dont tous les éléments de retenue seraient non dégradés (c'est-à-dire qu'ils seraient tous comme deux des zones de retenue). Cette souplesse accrue favorise la qualité de la retenue en permettant par exemple au dispositif de mieux accompagner les mouvements de la personne qui porte un article équipé d'un tel dispositif de retenue.

En particulier, dans chaque zone de retenue, la valeur de la première dimension géométrique des éléments de retenue varie dans une plage déterminée par rapport à une valeur maximale en étant par exemple comprise entre 80% et 100% de la valeur maximale, voire entre 85% et 100% de la valeur maximale, voire entre 90% et 100% de cette valeur maximale.

En particulier, la valeur maximale de la première dimension géométrique peut être sensiblement la même pour les deux zones de retenue, ce qui signifie que cette valeur maximale est la même pour les deux zones de retenue avec une tolérance de l'ordre de 10% voire 5%.

Ci-après, la « valeur maximale de référence » pour la première dimension géométrique est la plus grande des deux valeurs maximales de la première dimension géométrique respectivement constatées pour chacune des deux zones de retenue. Comme indiqué, ces deux valeurs maximales sont normalement proches, de sorte que valeur maximale de référence est elle-même proche de chacune de ces deux valeurs maximales.

En revanche, dans la zone intermédiaire, la valeur de la première dimension géométrique peut être nettement plus faible. Ainsi, pour au moins une partie des éléments de retenue de la zone intermédiaire, la valeur de la première dimension géométrique peut être inférieure ou égale à 30% de la valeur maximale de référence, voire inférieure ou égale à 50% de la valeur maximale de référence, voire inférieure ou égale à 60% de la valeur maximale de référence. Ici, « au moins une partie des éléments de retenue de la zone intermédiaire » signifie au moins un élément de retenue, voire au moins 10% ou au moins 20% ou au moins 40% des éléments de retenue de la zone intermédiaire.

Cependant, au moins une partie des éléments de retenue de la zone intermédiaire peuvent avoir des valeurs conséquentes pour la première dimension géométrique. Ainsi, au moins une partie des éléments de retenue de la zone intermédiaire peuvent présenter une valeur pour la première dimension géométrique de l'ordre d'au moins 5%, voire au moins 10%, voire au moins 20% de la valeur maximale de référence. Ici, « au moins une partie des éléments de retenue de la zone intermédiaire » signifie au moins un élément de retenue, voire au moins 10% ou au moins 20% ou au moins 40% des éléments de retenue de la zone intermédiaire.

Selon un deuxième aspect, l'exposé concerne un article absorbant du type couche-culotte pour bébé ou couche d'incontinence pour adulte, l'article comprenant un ensemble qui comprend deux feuilles extérieures et une âme absorbante disposée entre les feuilles extérieures, l'ensemble étant agencé de façon à présenter une première face de couche, en particulier une face avant, et des côtés de couche, l'article comprenant un système de retenue auto-agrippante comprenant des boucles de réception portées par l'un des éléments parmi la première face de couche et l'un des côtés de couche, et au moins un dispositif de retenue selon l'exposé, porté par l'autre des éléments parmi la première face de couche et ledit un des côtés de couche, de sorte que les éléments de retenue coopèrent avec les boucles de réception lorsque ledit au moins l'un des côtés de couche est placé contre la première face de couche pour retenir ledit au moins un des côtés de couche vis-à-vis de ladite première face.

La première face de couche peut en particulier être la face avant de la couche, c'est-à-dire la face externe située du côté du bas ventre de la personne qui la porte. Les côtés de couche peuvent être des pans latéraux de la couche ou des oreilles, en particulier des oreilles élastiques.

Par « retenir ledit au moins un des côtés de couche vis-à-vis de ladite première face », on entend fermer ce côté de la couche contre la première face en les retenant donc ensemble pour éviter qu'ils ne se séparent, et/ou retenir ce côté de couche contre la première face en évitant ou au moins en limitant leur glissement relatif. Optionnellement, le dispositif de retenue peut-être agencé sur une surface comprenant des boucles, par exemple sur une région de la bande confort (désignée généralement en anglais par « landing zone »), par exemple à proximité des bordures latérales de la bande confort.

Selon un troisième aspect, l'exposé concerne un procédé de fabrication d'un dispositif de retenue tel que défini précédemment, dans lequel :
- on fournit un dispositif de moulage présentant une pluralité de cavités formées en creux à partir d'une surface, le dispositif de moulage étant optionnellement une bande de moulage,
- on applique une matière de moulage chauffée sur ladite surface, à l'aide d'un applicateur en permettant à la matière de moulage de pénétrer dans les cavités pour former des éléments de retenue,
procédé selon lequel on génère deux flux adjacents séparés de matière de moulage que l'on applique sur la surface dans deux zones d'application en faisant fluer la matière de moulage pour que les deux flux se rejoignent dans une zone de jonction de manière à former une base, de sorte que la matière pénètre davantage dans les cavités présentes dans les zones d'application que dans les cavités présentes dans la zone de jonction.

Ici, « deux flux adjacents » signifie « au moins deux flux adjacents ». On peut en particulier avoir trois flux adjacents deux à deux, et deux zones de jonctions, chacune entre deux flux adjacents.

Optionnellement, l'applicateur est un dispositif d'extrusion comprenant deux canaux adjacents séparés par une cloison, et on applique la matière de moulage en déplaçant l'un par rapport à l'autre le dispositif de moulage et le dispositif d'extrusion dans une direction longitudinale.

Optionnellement les canaux peuvent être de sections identiques, la section d'un canal étant considérée transversalement au sens d'avancée de la matière dans le canal, c'est-à-dire étant normalement considérée selon la direction CD.

Il peut y avoir plus de deux canaux, séparés deux à deux par des cloisons respectives, qui peuvent optionnellement être identiques.

Optionnellement, les canaux peuvent être de sections différentes.

Optionnellement, les cloisons peuvent présenter des caractéristiques géométriques différentes. En particulier lorsque le nombre de canal est supérieur ou égal à 3, on peut concevoir que les canaux situés à l'extérieur dans la direction CD aient des sections différentes, en particulier plus faibles, que les canaux situés à l'intérieur. Optionnellement, les canaux et la ou les cloisons qui les séparent peuvent être disposés de manière symétrique par rapport à un plan de symétrie, qui est en particulier un plan défini par la direction MD et une direction perpendiculaire au plan défini par les directions MD et CD, ce plan de symétrie passant en particulier par le milieu de la largeur de l'applicateur.

Optionnellement, avant refroidissement de la matière de moulage, on applique un substrat contre la matière plastique appliquée sur la surface du dispositif de moulage, de sorte que ladite matière soit prise en sandwich entre la surface du dispositif de moulage et le substrat.

Bien entendu, on peut prévoir d'amener la même matière de moulage, ou au contraire des matières différentes, dans les différents canaux.

En particulier, les cavités du dispositif de moulage qui servent à former les éléments de retenue peuvent être toutes analogues.

En particulier, les cavités du dispositif de moulage qui servent à former les éléments de retenue peuvent être distribuées de manière homogène dans le dispositif de moulage (plus précisément, dans la surface du dispositif de moulage qui est destinée à recevoir la matière de moulage). Dans ce cas, ces cavités sont présentes dans les zones d'application et dans la zone de jonction selon la même distribution ou densité. Du fait du fluage mobilisant la matière dans la zone de jonction, cette matière présente moins de facilité à pénétrer dans les cavités présentes dans la zone de jonction que dans celles qui sont présentes dans les zones d'application, de sorte que des éléments de retenue issus des cavités présentes dans la zone de jonction sont dégradés par rapport à ceux qui sont issus des cavités présentes dans les zones d'application. Les zones d'applications servent donc à former des zones de retenue du dispositif de retenue, tandis que la zone de jonction sert à former une zone intermédiaire.

De fait du fluage dans la zone de jonction, la matière peut être présente dans cette zone en une épaisseur moindre que dans les zones d'application, de sorte que, dans sa partie issue de la zone de jonction, la base du dispositif de retenue peut avoir une épaisseur plus faible dans ses parties issues des zones d'application.

### Brève description des dessins

D'autres caractéristiques et avantages de l'objet du présent exposé ressortiront de la description suivante d'un mode de réalisation, donné à titre d'exemple non limitatif, en référence aux dessins annexés.
[Fig. 1] La figure 1 représente schématiquement un appareillage pour la réalisation d'un dispositif de retenue à éléments de retenue.
[Fig. 2] La figure 2 est un agrandissement de la zone II de la figure 1.
[Fig. 3] La figure 3 est une vue schématique en coupe selon la ligne III-III de la figure 1.
[Fig. 4] La figure 4 est une vue partielle schématique selon la flèche IV et la ligne IV-IV de la figure 1.
[Fig. 5] La figure 5 montre, en coupe transversale, un article comprenant un dispositif de retenue selon le présent exposé.
[Fig. 6] La figure 6 est une vue de dessus de l'article de la figure 5.
[Fig. 7] La figure 7 est une vue agrandie du détail VII de la figure 5.
[Fig. 8] La figure 8 est une vue partielle selon la flèche VIII de la figure 7.
[Fig. 9] La figure 9 est une de détail d'un élément de retenue du dispositif de retenue selon le présent exposé.
[Fig. 10] La figure 10 montre un article absorbant comprenant un dispositif de retenue selon l'exposé.

### Description détaillée

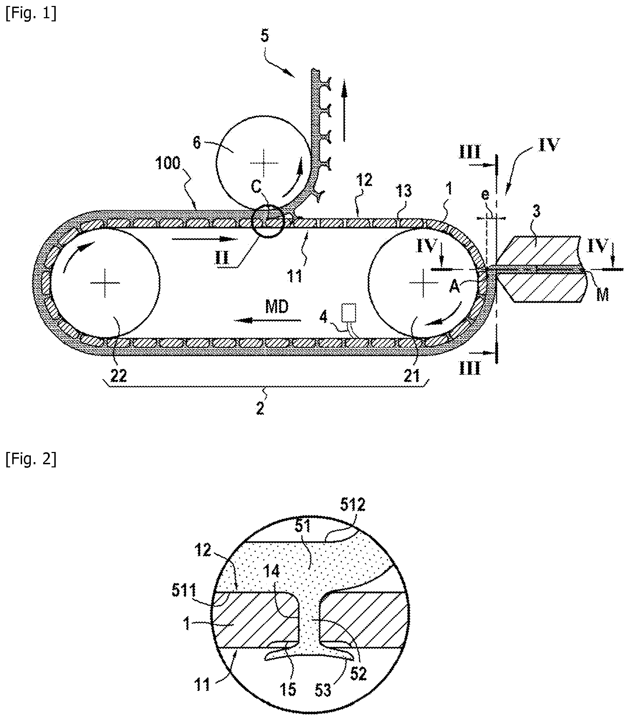

La figure 1 présente schématiquement un exemple d'appareillage pour la réalisation d'un dispositif de retenue à éléments de retenue.

L'appareillage tel que représenté comprend une bande de moulage 1 positionnée sur des moyens d'entrainement en rotation 2 comprenant ici deux rouleaux 21 et 22, et un moyen de distribution de matière ou applicateur 3 adapté pour appliquer une matière de moulage par exemple plastique et/ou élastique, sur une surface du dispositif de moulage.

La bande de moulage 1 est un exemple de dispositif de moulage.

L'appareillage sert à fabriquer un dispositif de retenue 5, qui est démoulé du dispositif de moulage à l'aide d'un rouleau 6.

L'exemple illustré est en l'espèce du type décrit dans le document WO2017187097 ; il peut notamment être modifié ou complété comme indiqué dans ce document ou dans le document FR 1914162. L'exemple illustré comprenant deux rouleaux 21, 22 n'est pas limitatif, le nombre et l'agencement du ou des rouleaux peuvent varier notamment afin de s'adapter à la longueur de la bande de moulage 1 et aux différents postes de l'appareillage. On pourrait par exemple utiliser trois rouleaux ou encore un seul de telle sorte que la bande de moulage est agencée sur la périphérie du seul rouleau pour former un manchon ou « sleeve » ou un « screen » selon les désignations en langue anglaise communément employées. En particulier, un seul des deux rouleaux peut être entrainé en rotation par des moyens motorisés, par exemple le rouleau 21, l'autre rouleau 22 étant libre, c'est à dire sans moyens motorisés, et entrainé en rotation via la bande de moulage, elle-même entrainée par le rouleau 21.

On définit une direction longitudinale par rapport à la direction d'avancement de la bande de moulage 1. Cette direction longitudinale est communément désignée par « direction machine » ou « machine direction » ou « MD » selon l'appellation en langue anglaise. On désigne la direction longitudinale par l'axe MD sur les figures.

On définit également une direction transverse, ou « cross direction » ou « CD » selon l'appellation en langue anglaise, correspondant à une direction perpendiculaire à la direction longitudinale, et s'étendant parallèlement aux faces interne et externe de la bande de moulage. On désigne la direction transverse par l'axe CD sur les figures.

La bande de moulage 1 telle que présentée comprend une face interne 11 et une face externe 12, la face interne 11 étant au contact des moyens d'entrainement en rotation 2, tandis que la face externe présente la surface sur laquelle la matière de moulage est appliquée par l'applicateur 3.

Plus précisément, l'applicateur 3 est disposé en regard de la bande de moulage 1, en étant espacé de la bande de moulage 1 de manière à définir un entrefer e indiqué sur la figure 1. On repère par la référence A la limite de la matière injectée sur la face externe 12 de la bande de moulage 1, correspondant au front arrière de la matière injectée sur la bande de moulage 1 par rapport au sens de déplacement de la bande de moulage 1.

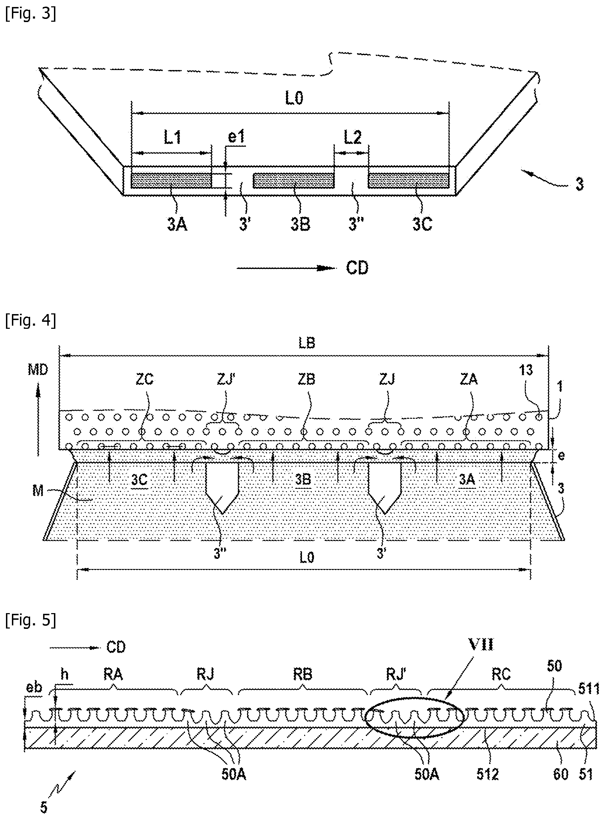

Comme on le voit également sur les figures 1 et 4, la bande de moulage 1 est munie d'une pluralité de cavités 13 permettant la réalisation d'éléments de retenue du dispositif de retenue 5.

En l'espèce, les cavités 13 représentées sur la figure 1 sont chacune formées de manière à servir à la réalisation d'éléments de retenue du type à crochets. Ainsi, comme on le voit mieux sur la figure 2, chaque cavité définit une tige 14 s'étendant depuis la face externe 12 vers la face interne 11 de la bande de moulage 1 et une tête 15 s'étendant entre la tige 14 et la face interne 11 de la bande de moulage 1.

Dans l'exemple illustré, les têtes 15 des cavités 13 débouchent sur la face interne 11 de la bande de moulage 1. Les cavités 13 sont donc traversantes. Un tel mode de réalisation n'est pas limitatif, les cavités 13 peuvent également être borgnes, et donc ne pas déboucher de la face interne 11 de la bande de moulage 1. De plus, les cavités peuvent être de formes différentes, notamment en étant dépourvues de têtes.

Les portions des cavités 13 formant les tiges 14 s'étendent typiquement selon une direction perpendiculaire à la face externe 12 de la bande de moulage 1. Les portions des cavités 13 formant les tiges 14 ont typiquement une géométrie de rotation autour d'un axe perpendiculaire à la face externe 12 de la bande de moulage 1, ou une géométrie présentant un plan de symétrie s'étendant selon une direction parallèle au sens de défilement de la bande de moulage 1 et/ou selon une direction perpendiculaire au sens de défilement de la bande de moulage 1.

Les portions des cavités 13 formant les tiges 14 présentent par exemple une forme généralement tronconique ou cylindrique de rotation autour d'un axe perpendiculaire à la face externe 12 de la bande de moulage 1, et présentant un arrondi au niveau de la jonction avec la face externe 12 de la bande de moulage 1.

Les portions des cavités 13 formant les têtes 15 s'étendent typiquement radialement ou transversalement par rapport à un axe perpendiculaire à la face externe 12 de la bande de moulage 1, et peuvent présenter une symétrie de rotation autour de cet axe perpendiculaire à la face externe 12 de la bande de moulage 1. Les portions des cavités 13 formant les têtes 15 présentent typiquement une forme tronconique ou hexaédrique, ou sensiblement tronconique ou hexaédrique.

Les portions des cavités 13 formant les têtes 15 peuvent être linéaires ou incurvées, par exemple former des portions incurvées vers la face interne 11 ou vers la face externe 12 de la bande de moulage 1 s'étendant depuis les portions des cavités 13 formant les tiges 14.

Les portions des cavités 13 formant les têtes 15 peuvent présenter une épaisseur constante ou variable.

Dans l'exemple représenté sur les figures, les portions des cavités 13 formant les têtes 15 s'étendent radialement autour des portions des cavités 13 formant les tiges 14, et présentent une forme générale de disque, comme on le voit notamment sur la figure 2 que l'on présentera par la suite.

La bande de moulage 1 peut présenter sur sa face interne 11 ou sur sa face externe 12 une texturation particulière telle que des rainures, réseau de gorges ou réseau de passage formant évent ou picots, ou être lisse ou sensiblement lisse.

La bande de moulage 1 peut être formée par une superposition de plusieurs bandes, et n'est donc pas nécessairement monobloc ou monomatière.

La bande de moulage peut présenter, dans la direction transversale CD, une largeur comprise entre 5 et 3 000 mm.

Le dispositif de moulage comprenant une bande de moulage qui vient d'être décrit est un exemple de dispositif de moulage. D'autres types de dispositifs pourraient être prévus, par exemple du type comprenant des plaques munies de cavités de moulage, ces plaques pouvant par exemple défiler en pas-à-pas.

On peut encore prévoir un autre type de dispositif de moulage, par exemple du type comprenant des rouleaux dans lesquels sont directement réalisées les cavités de moulage. Il peut s'agir de rouleaux massifs, usinés pour présenter les cavités de moulage, ou bien de disques empilés pour former un rouleau, les cavités de moulage étant formées par usinage des bords des disques et/ou par découpe des bords des disques, par exemple par laser, ou jet d'eau ou encore par électroérosion, en particulier électroérosion par fil. Bien entendu, les disques peuvent être empilés en faisant alterner des disques pleins aux bords parfaitement circulaires ou cylindriques, avec des disques dont les bords présentent des découpes.

La figure 2 représente la matière de moulage une fois injectée dans la bande de moulage 1. On représente sur la figure 2 une vue de côté (en coupe) de la matière dans les cavités 13 de la bande de moulage 1.

Comme on le voit sur la figure 2, la matière de moulage pénètre dans la bande de moulage de manière à remplir la cavité 13, formant ainsi une ébauche d'éléments de retenue, en l'espèce une ébauche de tige et de tête pour des éléments de retenue à crochets.

Une couche de matière de moulage est également déposée sur la face externe 12 de la bande de moulage 1 de manière à former une base pour le dispositif de retenue, l'épaisseur de cette couche de matériau de moulage étant déterminée par l'entrefer e entre la sortie de l'applicateur 3 et la bande de moulage 1.

L'entrefer e présente typiquement une épaisseur inférieure à 700 micromètres, ou typiquement entre 5 et 500 micromètres, ou encore entre 8 et 100 micromètres.

Dans l'exemple représenté, les cavités 13 de la bande de moulage 1 sont traversantes. L'appareillage peut alors comprendre un élément tel qu'une racle 4 positionné de manière à racler la face interne 11 de la bande de moulage 1 pour retirer au besoin le matériau de moulage excédentaire.

L'injection de matière de moulage dans la bande de moulage 1 par l'applicateur 3 permet donc de former des éléments de retenue dans les cavités 13, l'ensemble formant ainsi un ruban 100. Il peut s'agir d'éléments de retenue finis ou de préformes qui seront ensuite soumises à une étape de formage ou de calandrage pour leur finalisation, comme évoqué dans la demande de brevet WO2017187097. On entend ici par injection, l'action de mise en forme d'une matière de moulage par voie fondue, par exemple, la distribution, l'apport, le moulage, l'injection, l'extrusion.

En référence aux figures 3 et 4, on décrit maintenant l'applicateur 3. Cet applicateur est en l'espèce un dispositif d'extrusion qui comprend au moins deux canaux adjacents, séparés par une cloison. Dans l'exemple représenté, l'applicateur 3 comprend trois canaux 3A, 3B et 3C alignés, le canal 3B étant situé entre les canaux 3A et 3C, dont il est séparé par des cloisons 3' et 3".

Par exemple, la largeur L0 d'application de l'applicateur est comprise entre 70% et 100% de la largeur utile LB de la bande 1. Ici, la notion de « largeur d'application » signifie la distance transversale, mesurée selon la direction CD, entre les bords les plus éloignés des canaux d'application. Elle est ainsi mesurée entre un bord latéral externe du canal 3A et le bord latéral externe opposé du canal 3C. Les trois canaux 3A, 3B et 3C présentent en l'espèce la même largeur L1, qui est par exemple comprise entre 1 et 60mm, dans certains cas entre 2mm et 50mm, en particulier entre 3mm et 30mm. Cette largeur L1 est mesurée à la sortie des canaux. Les cloisons 3' et 3" ont en l'espèce la même largeur L2, également mesurée sur la face de sortie de l'applicateur, qui est par exemple comprise entre 0,5mm et 15mm, en particulier entre 0,5mm et 10mm. De préférence, la largeur L2 est inférieure ou égale à la largeur L1.

Les sorties des canaux 3A, 3B, 3C présentent en l'espèce la forme d'ouvertures rectangulaires de largeur L1 et de hauteur e1. Le ratio e1/L1 est en général inférieur ou égal à 2, voire inférieur ou égal à 1, voire même, comme dans l'exemple représenté, inférieur ou égal à 0,5.

Les sorties des canaux pourraient avoir des formes différentes, par exemple en étant carrée, circulaire, ovale, elliptique, ou encore en forme d'os de chien, c'est-à-dire une forme globalement rectangulaire, mais avec les bouts renflés en formant un ou deux lobes.

Sur la figure 4, les canaux 3A, 3B et 3C sont représentés en coupe schématique prise dans le plan IV-IV d'avancement de la matière de moulage, tandis que la bande de moulage est représentée en vue extérieure selon la flèche IV. On voit que, à la sortie des canaux, sont formés trois flux séparés de matière de moulage, 3A, 3B et 3C. Ces trois flux appliquent dont la matière de moulage sur la surface 12 de la bande 1 dans trois zones d'application ZA, ZB et ZC, qui les zones de la surface situées au droit des sorties des canaux, dans la direction d'avancement de la matière de moulage dans les canaux, qui correspond à la direction MD.

L'application de la matière de moulage sortie des canaux forme des rubans de matière de moulage.

On voit que, à la sortie des canaux et/ou une fois appliquée sur la surface 12, la matière de moulage flue latéralement (dans la direction CD). En effet, une fois appliquée sur la bande 1, la matière a naturellement tendance à s'étaler latéralement pour combler l'espace entre deux zones d'application adjacente. Du fait de la faible largeur L2 des cloisons, les flux adjacents vont avoir naturellement tendance à se rejoindre. Ainsi, les zones de la bande 1 situées au droit des cloisons 3' et 3" forment des zones de jonction ZJ, ZJ' dans lesquelles, du fait du fluage latéral de la matière de moulage, les rubans de matière de moulage appliqués en sortie des canaux 3A, 3B et 3C ont tendance à se rejoindre. Comme on l'a indiqué sur la figure 4 par les références f, le phénomène de fluage latéral peut prendre effet dès la sortie des canaux, dans l'entrefer e, éventuellement avant que la matière de moulage ne parvienne au contact de la surface 12.

La matière de moulage directement appliquée dans les zones d'application ZA, ZB et ZC, par son avancement dans le sens MD va naturellement remplir les cavités 13 présentes dans ces zones, sous l'effet de sa composante de vitesse et des pressions de sortie des canaux, qui s'exercent alors principalement perpendiculairement au plan de la surface 12. En revanche, dans les zones de jonction ZJ et ZJ", cette pression est en partie consacrée au fluage latéral et sa composante de vitesse s'exerce naturellement et principalement selon la direction CD. Du fait de son étalement latéral, la matière de moulage a moins tendance à remplir les cavités présentes dans les zones de jonction. Ainsi, les parties du dispositif de retenue issues de ces zones de jonction présentent des éléments de retenues qui sont dégradés par rapport à ceux des parties issues de zones d'application, alors que les cavités 13 des zones de jonction et des zones d'application sont identiques ou analogues.

C'est ce que l'on voit sur la figure 5. Cette figure montre un dispositif de retenue qui comprend une base 51 et des éléments de retenue 50 qui s'étendent de la face supérieure 511 de la base 51. En l'espèce, le dispositif de retenue comprend également un substrat 60 du côté de la face inférieure 512 de la base 51.

La face supérieure 511 et une face inférieure 512 de la base sont typiquement parallèles ou sensiblement parallèles, la face supérieure 511 étant la face munie des éléments de retenue 50.

On voit que le dispositif présente des zones de retenue RA, RB et RC, dans lesquelles les éléments de retenue sont standard. Ceci signifie que les éléments de retenue présents dans ces zones de retenue ont été en général correctement moulés dans les cavités. Les éléments de retenue de ces zones présentent tous sensiblement la même hauteur h mesurée entre la face supérieure 511 de la base 51 et leur extrémité supérieure opposée. Ceci signifie notamment que les hauteurs de ces éléments de retenue sont toutes entre 80% et 100%, voire entre 85% et 100% et voire 90% et 100% de la hauteur maximale constatée pour ces éléments de retenue.

Ici, la dimension géométrique prise en compte est la hauteur des éléments de retenue. On pourrait prendre en compte d'autres dimensions géométriques, par exemple l'épaisseur des tiges des éléments de retenue mesurée dans un plan parallèle à la face supérieure de la base, ou entre la largeur des têtes des éléments de retenue (si ceux-ci sont pourvus de têtes), également mesurée dans un plan parallèle à la face supérieure de la base.

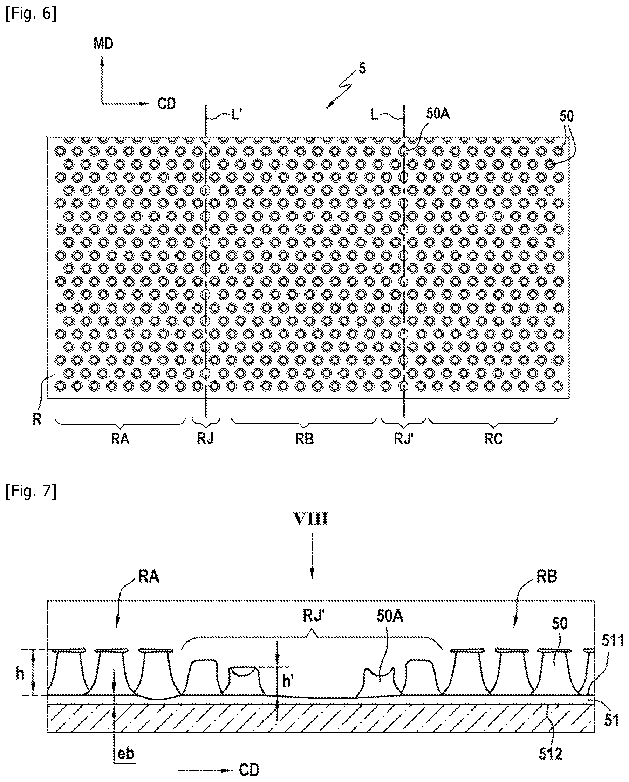

Entre les zones de retenue RA, RB et RC, le dispositif de retenue présente des zones intermédiaires RJ et RJ'. On voit que, parmi les éléments de retenue 50, les éléments de retenue 50A qui sont présents dans les zones intermédiaire RJ et RJ' sont dégradés par rapport aux autres. En particulier, on voit que leurs hauteurs h' sont plus faibles que celle des éléments de retenue présents dans les zones de retenue et même, éventuellement, que ces hauteurs varient assez nettement d'un élément de retenue 50A à l'autre. On voit même, notamment sur la figure 7 que les éléments de retenue 50A des zones intermédiaires RJ et RJ' peuvent être dépourvus de tête, contrairement à ceux des zones de retenue RA, RB et RC.

Là encore, la hauteur n'est que l'une des dimensions géométriques des éléments de retenue 50A, qui est prise en considération pour apprécier leur dégradation par rapport aux éléments de retenue qui sont présent dans les zones de retenue. On comprend donc ici que la différence de forme entre les éléments de retenue 50 des zones de retenue et des zones intermédiaires résulte non pas de géométries différentes des cavités 13 de la bande 1, mais d'un remplissage non uniforme de ces cavités par la matière de moulage entre les zones d'application et les zones de jonction.

Sur la figure 6, on voit la forme de rubans allongés selon la direction MD que présentent les zones de retenue RA, RB et RC, les zones intermédiaires RJ et RJ' ayant également la forme de rubans, en général moins larges (la largeur étant mesurée dans la direction CD). Ces zones intermédiaires présentent des lignes de soudure L et L', correspondant à la jonction des flux issus des canaux 3A et 3B d'une part, et 3B et 3C d'autre part. Dans les zones intermédiaires, la base peut présenter un état de matière différent de celui qu'elle présente dans les zones de retenue. En particulier, la base peut présenter une orientation moléculaire dans les zones de retenue qui est homogène et par exemple selon la direction MD, alors que l'orientation moléculaire peut varier dans les zones intermédiaires, en raison du fluage de la matière de moulage dans des directions ayant une composante non nulle selon la direction CD. On voit cependant que ces différentes zones forment un même ruban commun R, les zones intermédiaires réalisant la jonction entre les zones de retenue.

Comme on le voit mieux sur la figure 6, les éléments de retenue 50 sont répartis selon un motif régulier et répétitif. Ici, ils sont disposés selon des lignes parallèles à la direction CD et des colonnes parallèles à la direction MD, les éléments de retenue des lignes et des colonnes adjacentes étant disposés en quinconce.

On voit d'ailleurs sur la figure 4 que les cavités du dispositif de moulage sont réparties selon ce motif régulier et répétitif.

Sur les figures 5 et 7, on voit que, en considérant une zone intermédiaire RJ dans le sens allant d'une zone de retenue RA à la zone de retenue adjacente RB, en particulier un sens selon la direction CD, la hauteur des éléments de retenue décroît vers une zone centrale de la zone intermédiaire, puis croît.

La base 51 présente typiquement une épaisseur eb comprise entre 3 et 500 micromètres, ou plus spécifiquement entre 4 et 150 micromètres, ou encore entre 4 et 120 micromètres, ou encore entre 4,5 et 108 micromètres.

Dans les zones de retenue RA, RB et RC, l'épaisseur de la base est constante ou sensiblement constante. Elle est mesurée entre deux éléments de retenue 50 adjacents des zones de retenue. Par « sensiblement constante », on entend que l'épaisseur varie tout au plus de 20%, voire 10%, par rapport à une valeur moyenne.

En revanche, l'épaisseur de la base 51 peut être diminuée dans les zones intermédiaires RJ et RJ', ainsi qu'on le voit sur les figures 5 et 7. Elle peut par exemple diminuer jusqu'à représenter 80%, voire 60%, voire encore 50% ou moins de l'épaisseur moyenne de la base dans les zones de retenue.

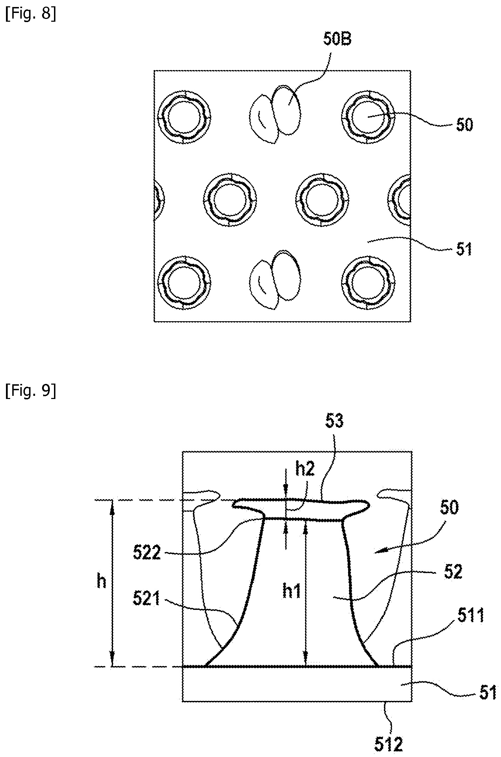

Comme on le voit sur la figure 8, il peut même arriver que la ou les zones intermédiaires PJ, RJ', présentent des vides 50B. Ceci correspond à des zones dans lesquelles, soit la jonction par fluage entre les deux zones d'application de la matière de moulage ne s'est localement pas opérée, soit la matière a insuffisamment pénétré dans les cavités de la bande de moulage et a formé des portions d'éléments de retenue en forme de cheminées, soit encore la matière a pénétré dans une cavité pour former un élément de retenue dégradé 50A, au détriment de son fluage latéral, laissant ainsi un vide au pied de cet élément de retenue dégradé. Les vides 50B présentent généralement de petites dimensions. En particulier, la dimension maximale d'un vide, par la longueur d'un segment allant d'un bord à l'autre du vide, parallèlement à la surface supérieure de la base, peut être inférieure à la distance minimale entre deux éléments de retenue.

La base 51 présente typiquement une largeur comprise entre 1 et 3 000 millimètres, ou plus précisément entre 2 et 400 millimètres, ou encore entre 3 et 100 millimètres, la largeur de la base 51 étant mesurée selon la direction transversale par rapport à la direction longitudinale, par exemple selon une direction parallèle à la face externe 12 de la bande de moulage 1. Cette largeur correspond à la largeur dite utile LB de la bande 1 du dispositif de moulage.

On décrit certaines caractéristiques géométrique d'un élément de retenue 50 non dégradé (c'est-à-dire présent dans une zone de retenue) en référence à la figure 9. Cet élément de retenue présente en l'occurrence une forme de crochet, avec une tige 52 et une tête 53, qui dépasse latéralement de la tige. La hauteur totale h de l'élément de retenue 50 peut être de l'ordre de 80 à 1000 micromètres, en particulier de 90 à 500 micromètres, en particulier de 90 à 450 micromètres. La hauteur h1 de la tige 52, mesurée depuis la face supérieure de la base 51 jusqu'à la face inférieure de la tête 53, peut être de l'ordre de 80 à 800 micromètres, en particulier de 90 à 450 micromètres ou encore de 90 à 300 micromètres. La hauteur h2 de la tête 53, mesurée entre ses faces inférieure et supérieure, entre deux plans parallèles à la face supérieure de la base et tangente auxdites faces inférieure et supérieure, peut être de l'ordre de 5 à 200 micromètres, en particulier de 10 à 100 micromètres.

Comme indiqué, l'ensemble formé par la base et les éléments de retenue peut présenter un grammage compris entre 10 et 120 g/m², en particulier entre 30 et 80 g/m², plus particulièrement entre 50 et 70 g/m².

La base en elle-même peut présenter un grammage compris entre 5 et 80 g/m², en particulier entre 10 et 60 g/m².

Les ratios suivants peuvent être constatés dans les zones de retenue :
- hauteur de la tige sur hauteur de la base : 0,8 à 80, en particulier 1,5 à 65 ;
- hauteur de la tête sur hauteur de la base : 0,1 à 30, en particulier 0,3 à 22 ;
- hauteur de la tige/grammage de l'ensemble formé par la base et les éléments de retenue : 1 à 10 (en micromètres /g/m²).

La hauteur de l'élément de retenue est mesurée par la longueur d'un segment de droite partant du centre de l'élément de retenue, au niveau de la surface supérieure la base (niveau moyen de cette surface vérifié entre deux éléments de retenue adjacents dans la zone concernée) et arrivant jusqu'à son point d'intersection avec l'enveloppe extérieure du crochet.

La base peut être formée d'une seule matière qui peut également être celle des éléments de retenue.

Le démoulage du dispositif de retenue est typiquement réalisé lorsque la base est à une température inférieure à la température de fusion du matériau de moulage, ou inférieure à la température de fléchissement sous charge du matériau de moulage, par exemple lorsque la face interne 11 de la bande de moulage 1 est à une température de l'ordre de 45°C et la face supérieure 511 de la base 51 est à une température de l'ordre de 75°C. La température de fléchissement sous charge est communément désignée par son appellation en langue anglaise « Heat Deflection Temperature » ou « HDT ».

La bande de moulage 1 est classiquement maintenue, dans la zone de moulage, à une température comprise entre HDT-30°C et HDT+10°C, en particulier entre 50°C et 120°C, en particulier de l'ordre de 80°C, notamment lorsque la matière de moulage est du polypropylène ou, de manière générale, un copolymère d'éthylène.

La base 51 pouvant être extrêmement fine, la température de la bande de moulage, dans la zone de moulage, peut être légèrement supérieure à HDT car la surface extérieure de la base (opposée à la bande), qui est en contact avec l'air est à une température inférieure à celle de la bande et, du fait de la faible épaisseur de la base, la face intérieure de la base et les éléments de retenue refroidissent dès que la base se sépare d'avec la bande de moulage.

L'étape de démoulage peut être suivie d'une étape de formage, dans laquelle les deuxièmes préformes sont modifiées notamment au niveau de leur tête 53.

Comme indiqué, le dispositif de retenue peut comprendre un substrat 60. Ce substrat est typiquement une couche de matériau non tissé, un film plastique, un film élastique ou un film composite, ou encore un ensemble de fibres et/ou filaments consolidé thermiquement. Le substrat 60 est par exemple une nappe de fibres et/ou filaments.

On entend par non tissé un produit obtenu à l'issue de la formation d'une nappe de fibres et/ou de filaments qui ont été consolidés. La consolidation peut être mécanique, chimique ou thermique et se traduit par la présence de liaison entre les fibres et/ou les filaments. Cette consolidation peut être directe, c'est-à-dire faite directement entre les fibres et/ou filaments par soudure, ou elle peut être indirecte, c'est-à-dire par l'intermédiaire d'une couche intermédiaire entre les fibres et/ou les filaments, par exemple une couche de colle ou une couche de liant. Le terme non-tissé se rapporte à une structure en forme de ruban ou nappe de fibres et/ou filaments qui sont entrelacées d'une manière non uniforme, irrégulière ou au hasard. Un non-tissé peut avoir une structure de couche unique ou une structure à couches multiples. Un non-tissé peut également être réuni à un autre matériau pour former un stratifié. Un non-tissé peut être réalisé à partir de différents matériaux synthétiques et/ou naturels. Les matériaux naturels à titre d'exemple sont des fibres de cellulose, telles que coton, jute, lin et analogue et peuvent également inclure des fibres de cellulose re-traitées, telles que la rayonne ou la viscose. Les fibres naturelles pour un matériau non-tissé peuvent être préparées en utilisant divers procédés tels que le cardage. Des matériaux synthétiques à titre d'exemple comportent, mais sans s'y limiter, des polymères thermoplastiques synthétiques, qui sont connus pour former des fibres qui incluent, sans s'y limiter, les polyoléfines, par exemple le polyéthylène, polypropylène, polybutylène et analogue; le polyamide, par exemple le polyamide 6, polyamide 6.6, polyamide 10, polyamide 12 et analogue; des polyesters, par exemple des polyéthylènes téraphthalates, des polybutylènes téréphtalates, des acides polylactiques et analogues, des polycarbonates, des polystyrènes, des élastomères thermoplastiques, des vinyles polymères, des polyuréthanes et des mélanges et des co-polymères de ces derniers. A titre d'exemple, le non tissé peut être un non tissé du type Spunbond, Spunmelt, cardé thermolié, SMS, SMMS, SS, SSS, SSMMS, SSMMMS, Air through ou autre.

Le substrat n'est pas limité à un non tissé, et peut plus généralement être un non tissé, un matériau tissé, un matériau tricoté, ou une combinaison de plusieurs de ces matériaux.

Dans le cas où le substrat 60 est un non tissé, il peut être activé préalablement à sa solidarisation à la base. Le substrat peut comprendre plusieurs couches distinctes, notamment une couche de support, qui peut elle-même être un non tissé et éventuellement être activée.

On entend par matériau plastique un matériau thermoplastique, plus particulièrement un matériau polyoléfine à base de d'homopolymère ou de copolymère.

Les matériaux mentionnés dans le document WO2017187097 peuvent être utilisés pour former la base et les éléments de retenue et, s'il est présent, le substrat. Le substrat peut être rapporté sur la base comme mentionné dans le document WO2017187097.

L'objet du présent exposé permet, tout en ayant d'excellente propriétés de retenue, éventuellement sur des largeurs conséquentes, de faire en sorte que le dispositif de retenue soit particulièrement léger. De plus, il est d'une grande souplesse, en particulier dans la ou les zones intermédiaires. Il constitue un tout, les zones de retenue étant jointives avec la ou les zones intermédiaires.

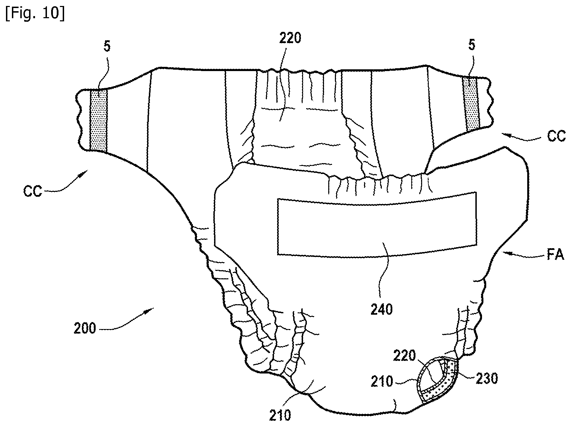

La figure 10 montre un article absorbant 200 du type couche-culotte pour bébé ou couche d'incontinence pour adulte. Typiquement, l'article 200 comprend un ensemble qui comprend deux feuilles extérieures 210, 220 et une âme absorbante 230 disposée entre les feuilles extérieures. L'article présente une première face de couche (face avant) FA, et des côtés de couche CC. L'article comprend un système de retenue auto-agrippante comprenant des boucles de réception portées par l'un des éléments parmi la première face de couche FA et l'un des côtés de couche CC. En l'espèce, sur la figure 10, le rectangle 240 matérialisé sur la première face de la couche FA comprend de telles boucles de réception et correspond à une bande confort (désignée généralement en anglais par « landing zone »).

L'article comprend également un dispositif de retenue 5 selon le présent exposé. En l'espèce, un tel dispositif 5 est disposé sur chacun des côtés CC de manière à pouvoir coopérer avec les boucles de réception 240 qui sont ici portées par la face avant FA, afin de maintenir fermé l'article porté par l'utilisateur.

## Revendications

1. Dispositif de retenue, comprenant
- une base (51) s'étendant selon une direction longitudinale (MD) et présentant une face supérieure (511) et une face inférieure (512),
- une pluralité d'éléments de retenue (50,50A) s'étendant de la face supérieure de la base, chaque élément de retenue présentant une valeur selon une première dimension géométrique, ladite valeur selon une première dimension géométrique étant l'une parmi :
- la hauteur des éléments de retenue (50, 50A), mesurée, pour chaque élément de retenue, entre une extrémité inférieure de l'élément de retenue reliée à la base (51) et une extrémité supérieure de l'élément de retenue (50, 50A) opposée à l'extrémité inférieure,
- une dimension mesurée selon une vue en coupe perpendiculaire à la base (51), en particulier une vue en coupe perpendiculaire à la base et parallèle à une direction transversale (CD),
- une dimension mesurée selon une vue parallèle à la base (51) et à distance des éléments de retenue (50, 50A),
- une dimension transversale (CD) des éléments de retenue (50, 50A), mesurée parallèlement à la face supérieure (511) de la base (51),
- une dimension diamétrale d'une portion des éléments de retenue (50, 50A) dans le cas où les éléments de retenue (50, 50A) présentent, au moins dans certaines portions, une symétrie de révolution,
- l'épaisseur d'une tige (52) des éléments de retenue (50, 50A) ou la largeur de têtes (53) des éléments de retenue (50, 50A), mesurée dans un plan parallèle à la face supérieure (511) de la base (51),
**caractérisé en ce que**
le dispositif de retenue présente au moins deux zones de retenue (RA, RB, RC) et une zone intermédiaire (RJ, RJ') disposée entre les deux zones de retenue et reliant les deux zones de retenue, les éléments de retenue (50) disposés dans les première et deuxième zones de retenue ayant des valeurs selon la première dimension géométrique (h) qui sont supérieures aux valeurs selon la première dimension géométrique des éléments de retenue (50A) disposés dans la zone intermédiaire, et **en ce que** l'ensemble formé par la base (51) et les éléments de retenue (50, 50A) présente un grammage compris entre 10 et 120 g/m².

2. Dispositif de retenue selon la revendication 1, dans lequel les zones de retenue (RA, RB, RC) et la zone intermédiaire (RJ, RJ') forment des zones allongées en forme de ruban dans un ruban commun (R), les zones allongées et le ruban commun étant optionnellement allongés selon la direction longitudinale (MD).

3. Dispositif de retenue selon la revendication 1 ou 2, dans lequel la zone intermédiaire (RJ, RJ') présente au moins une partie dans laquelle la base (51) présente une épaisseur inférieure à une épaisseur (eb) de la base dans les zones de retenue (RA, RB, RC).

4. Dispositif de retenue selon l'une des revendications 1 à 3, dans lequel, dans la zone intermédiaire (RJ, RJ'), la base (51) présente des vides (50B).

5. Dispositif de retenue selon l'une des revendications 1 à 4, dans lequel, dans les zones de retenue (RA, RB, RC), les éléments de retenue (51) présentent une valeur selon la première dimension géométrique (h) qui est sensiblement constante.

6. Dispositif de retenue selon l'une des revendications 1 à 5, dans lequel, considérée dans le sens allant de la première zone de retenue (RA) à la deuxième zone de retenue (RB), la zone intermédiaire (RJ) présente successivement des éléments de retenue (50A) dont la valeur selon la première dimension géométrique décroît puis des éléments de retenue dont la valeur selon la première dimension géométrique croît.

7. Dispositif de retenue selon l'une des revendications 1 à 6, dans lequel toute ligne droite passant par au moins un élément de retenue (50, 50A) et s'étendant selon la direction allant de la première zone de retenue (RA) à la deuxième zone de retenue (RB) en passant par lesdites première et deuxième zones de retenue, croise au moins 3 éléments de retenue, en particulier au moins 5 éléments de retenue dans chacune des première et deuxième zones de retenue et, optionnellement, au moins 1 élément de retenue, en particulier au moins 2 éléments de retenue dans la zone intermédiaire.

8. Dispositif de retenue selon l'une des revendications 1 à 7, dans lequel, au moins dans les zones de retenue (RA, RB, RC), les éléments de retenue (50) sont chacun formés d'une tige (52) surmontée d'une tête (53) dépassant de la tige, une partie au moins des éléments de retenue de la zone intermédiaire étant optionnellement dépourvus de tête.

9. Dispositif selon l'une des revendications 1 à 8, dans lequel l'ensemble formé par la base (51) et les éléments de retenue (50, 50A) présente un grammage compris entre entre 30 et 80 g/m², dans certain cas entre 30 et 70 g/m², plus particulièrement entre 50 et 70 g/m².

10. Dispositif selon l'une des revendications 1 à 9, comprenant en outre un substrat (60) portant la base, le substrat comprenant optionnellement une couche de matériau non-tissé.

11. Article absorbant (200) du type couche-culotte pour bébé ou couche d'incontinence pour adulte, l'article comprenant un ensemble qui comprend deux feuilles extérieures (210, 220) et une âme absorbante (230) disposée entre les feuilles extérieures, l'ensemble étant agencé de façon à présenter une première face de couche (FA), en particulier une face avant, et des côtés de couche (CC), l'article comprenant un système de retenue auto-agrippante comprenant des boucles de réception (240) portées par l'un des éléments parmi la première face de couche et l'un des côtés de couche, et au moins un dispositif de retenue (5) selon l'une des revendications 1 à 10, porté par l'autre des éléments parmi la première face de couche et ledit un des côtés de couche, de sorte que les éléments de retenue coopèrent avec les boucles de réception lorsque ledit au moins l'un des côtés de couche est placé contre la première face de couche pour retenir ledit au moins un des côtés de couche vis-à-vis de ladite première face.

12. Procédé de fabrication d'un dispositif de retenue tel que défini par la revendication 1, dans lequel :
- on fournit un dispositif de moulage (1) présentant une pluralité de cavités (13) formées en creux à partir d'une surface (12),
- on applique une matière de moulage (M) chauffée sur ladite surface, à l'aide d'un applicateur (3) en permettant à la matière de moulage de pénétrer dans les cavités (13) pour former des éléments de retenue,
**caractérisé en ce qu'**on génère deux flux adjacents séparés (FA, FB et/ou FC) de matière de moulage (M) que l'on applique sur la surface (12) dans deux zones d'application en faisant fluer la matière de moulage pour que les deux flux se rejoignent dans une zone de jonction de manière à former une base (51), de sorte que la matière pénètre davantage dans les cavités présentes dans les zones d'application que dans les cavités présentes dans la zone de jonction.

13. Procédé selon la revendication 12, dans lequel l'applicateur (3) est un dispositif d'extrusion comprenant deux canaux adjacents (3A, 3B et/ou 3C) séparés par une cloison (3', 3"), et on applique la matière de moulage (M) en déplaçant l'un par rapport à l'autre le dispositif de moulage (1) et le dispositif d'extrusion (3) dans une direction longitudinale.

14. Procédé selon l'une des revendications 12 à 13, dans lequel, avant refroidissement de la matière de moulage (M), on applique un substrat (60) contre la matière plastique appliquée sur la surface du dispositif de moulage (1), de sorte que ladite matière soit prise en sandwich entre la surface du dispositif de moulage et le substrat.

## Patentansprüche

1. Rückhaltevorrichtung, umfassend
- eine Basis (51), die sich gemäß einer Längsrichtung (MD) erstreckt und eine obere Seite (511) und eine untere Seite (512) aufweist,
- mehrere Rückhalteelemente (50, 50A), die sich von der oberen Seite der Basis weg erstrecken, wobei jedes Rückhalteelement einen Wert gemäß einer ersten geometrischen Dimension aufweist, wobei der Wert gemäß einer ersten geometrischen Dimension einer der folgenden ist:
- die Höhe der Rückhalteelemente (50, 50A), die für jedes Rückhalteelement zwischen einem unteren Ende des Rückhalteelements, das mit der Basis (51) verbunden ist, und einem oberen Ende des Rückhalteelements (50, 50A), das dem unteren Ende gegenüberliegt, gemessen wird,
- eine Dimension, die gemäß einer Schnittansicht senkrecht auf die Basis (51), insbesondere einer Schnittansicht senkrecht auf die Basis und parallel zu einer Querrichtung (CD) gemessen wird,
- eine Dimension, die gemäß einer Ansicht parallel zu der Basis (51) und in einem Abstand zu den Rückhalteelementen (50, 50A) gemessen wird,
- eine querverlaufende Dimension (CD) der Rückhalteelemente (50, 50A), die parallel zu der oberen Seite (511) der Basis (51) gemessen wird,
- eine diametrale Dimension eines Abschnitts der Rückhalteelemente (50, 50A) in dem Fall, in dem die Rückhalteelemente (50, 50A) zumindest in bestimmten Abschnitten eine Rotationssymmetrie aufweisen,
- die Dicke eines Schafts (52) der Rückhalteelemente (50, 50A) oder die Breite der Köpfe (53) der Rückhalteelemente (50, 50A), die in einer Ebene parallel zu der oberen Seite (511) der Basis (51) gemessen wird,
**dadurch gekennzeichnet, dass**
die Rückhaltevorrichtung zumindest zwei Rückhaltezonen (RA, RB, RC) und eine Zwischenzone (RJ, RJ') aufweist, die zwischen den zwei Rückhaltezonen angeordnet ist und die zwei Rückhaltezonen verbindet, wobei die Rückhalteelemente (50), die in der ersten und zweiten Rückhaltezone angeordnet sind, Werte gemäß der ersten geometrischen Dimension (h) aufweisen, die größer sind als die Werte gemäß der ersten geometrischen Dimension der Rückhalteelemente (50A), die in der Zwischenzone angeordnet sind,
und dass die Anordnung, die aus der Basis (51) und den Rückhalteelementen (50, 50A) gebildet wird, ein Flächengewicht zwischen 10 und 120 g/m² aufweist.

2. Rückhaltevorrichtung nach Anspruch 1, wobei die Rückhaltezonen (RA, RB, RC) und die Zwischenzone (RJ, RJ') längliche Zonen in Bandform in einem gemeinsamen Band (R) bilden, wobei die länglichen Zonen und das gemeinsame Band optional gemäß der Längsrichtung (MD) länglich sind.

3. Rückhaltevorrichtung nach Anspruch 1 oder 2, wobei die Zwischenzone (RJ, RJ') zumindest einen Teil aufweist, in welchem die Basis (51) eine Dicke aufweist, die geringer ist als eine Dicke (eb) der Basis in den Rückhaltezonen (RA, RB, RC).

4. Rückhaltevorrichtung nach einem der Ansprüche 1 bis 3, wobei die Basis (51) in der Zwischenzone (RJ, RJ') Leerstellen (50B) aufweist.

5. Rückhaltevorrichtung nach einem der Ansprüche 1 bis 4, wobei die Rückhalteelemente (51) in den Rückhaltezonen (RA, RB, RC) einen Wert gemäß der ersten geometrischen Dimension (h) aufweisen, der im Wesentlichen konstant ist.

6. Rückhaltevorrichtung nach einem der Ansprüche 1 bis 5, wobei, in der Richtung betrachtet, die von der ersten Rückhaltezone (RA) zu der zweiten Rückhaltezone (RB) verläuft, die Zwischenzone (RJ) der Reihe nach Rückhalteelemente (50A), deren Wert gemäß der ersten geometrischen Dimension ansteigt, und dann Rückhalteelemente aufweist, deren Wert gemäß der ersten geometrischen Dimension absinkt.

7. Rückhaltevorrichtung nach einem der Ansprüche 1 bis 6, wobei jede gerade Linie, die durch zumindest ein Rückhalteelement (50, 50A) verläuft und sich gemäß der Richtung erstreckt, die von der ersten Rückhaltezone (RA) zu der zweiten Rückhaltezone (RB) und dabei durch die erste und zweite Rückhaltezone verläuft, zumindest 3 Rückhalteelemente, insbesondere zumindest 5 Rückhalteelemente in jeder der ersten und zweiten Rückhaltezonen kreuzt, und optional zumindest 1 Rückhalteelement, insbesondere zumindest 2 Rückhalteelemente in der Zwischenzone.

8. Rückhaltevorrichtung nach einem der Ansprüche 1 bis 7, wobei zumindest in den Rückhaltezonen (RA, RB, RC) die Rückhalteelemente (50) jeweils aus einem Schaft (52) gebildet sind, auf dem ein Kopf (53) aufgebracht ist, der über den Schaft hinausragt, wobei zumindest ein Teil der Rückhalteelemente der Zwischenzone optional nicht mit einem Kopf versehen ist.

9. Vorrichtung nach einem der Ansprüche 1 bis 8, wobei die Anordnung, die aus der Basis (51) und den Rückhalteelementen (50, 50A) gebildet wird, ein Flächengewicht zwischen 30 und 80 g/m², in bestimmten Fällen zwischen 30 und 70 g/m², insbesondere zwischen 50 und 70 g/m² aufweist.

10. Vorrichtung nach einem der Ansprüche 1 bis 9, ferner umfassend ein Substrat (60), das die Basis trägt, wobei das Substrat optional eine Schicht aus Vliesmaterial umfasst.

11. Absorbierender Artikel (200) vom Typ Babywindel oder Inkontinenzwindel für Erwachsene, wobei der Artikel eine Anordnung umfasst, die zwei äußere Lagen (210, 220) und einen absorbierenden Kern (230) umfasst, der zwischen den äußeren Lagen angeordnet ist, wobei die Anordnung auf solche Weise angeordnet ist, dass sie eine erste Windel-Seitenfläche (FA), insbesondere eine Stirnfläche, und Windel-Randseiten (CC) aufweist, wobei der Artikel ein Klett-Rückhaltesystem, dass Aufnahmeschleifen (240) umfasst, die von einem der Elemente aus der ersten Windel-Seitenfläche und einer der Windel-Randseiten getragen werden, und zumindest eine Rückhaltevorrichtung (5) nach einem der Ansprüche 1 bis 10 umfasst, die von dem anderen der Elemente aus der ersten Windel-Seitenfläche und der einen der Windel-Randseiten getragen wird, auf solche Weise, dass die Rückhalteelemente mit den Aufnahmeschleifen zusammenwirken, wenn die zumindest eine der Windel-Randseiten gegen die erste Windel-Seitenfläche platziert wird, um die zumindest eine der Windel-Randseiten der ersten Seitenfläche gegenüber zurückzuhalten.

12. Verfahren zur Herstellung einer Rückhaltevorrichtung wie in Anspruch 1 definiert, wobei:
- eine Formvorrichtung (1) bereitgestellt wird, die mehrere Hohlräume (13) aufweist, die ausgehend von einer Oberfläche (12) vertieft ausgebildet sind,
- ein erwärmtes Formmaterial (M) mithilfe einer Auftragsvorrichtung (3) auf die Oberfläche aufgetragen wird, wobei ermöglicht wird, dass das Formmaterial in die Hohlräume (13) eindringen kann, um Rückhalteelemente auszubilden,
**dadurch gekennzeichnet, dass** zwei benachbarte getrennte Ströme (FA, FB und/oder FC) des Formmaterials (M) erzeugt werden, dass dieses auf die Oberfläche (12) in zwei Auftragszonen aufgetragen wird, indem das Formmaterial so fließen gelassen wird, dass die zwei Ströme sich in einer Vereinigungszone auf solche Weise vereinigen, dass eine Basis (51) gebildet wird, auf eine Weise, dass das Material in die Hohlräume, die in den Auftragszonen vorhanden sind, tiefer eindringt als in die Hohlräume, die in der Vereinigungszone vorhanden sind.

13. Verfahren nach Anspruch 12, wobei die Auftragsvorrichtung (3) eine Extrusionsvorrichtung ist, die zwei benachbarte Kanäle (3A, 3B und/oder 3C) umfasst, die durch eine Trennwand (3', 3") getrennt sind, und das Formmaterial (M) aufgetragen wird, indem eine von der Formvorrichtung (1) und der Extrusionsvorrichtung (3) in Bezug auf die andere in einer Längsrichtung verschoben wird.

14. Verfahren nach einem der Ansprüche 12 bis 13, wobei vor der Abkühlung des Formmaterials (M) ein Substrat (60) gegen das Kunststoffmaterial, das auf die Oberfläche der Formvorrichtung (1) aufgetragen wurde, auf eine Weise aufgebracht wird, dass das Material sandwichartig zwischen der Oberfläche der Formvorrichtung und dem Substrat angeordnet ist.

## Claims

1. A retainer device, comprising:
- a base (51) extending along a longitudinal direction (MD) and having an upper face (511) and a lower face (512),
- a plurality of retainer elements (50, 50A) extending from the upper face of the base, each retainer element having a value according to a first geometric dimension, said value according to a first geometric dimension being one among:
- the height of the retainer elements (50, 50A), measured, for each retainer element, between a lower end of the retainer element connected to the base (51) and an upper end of the retainer element opposite to the lower end,
- a dimension measured according to a sectional view perpendicular to the base (51), particularly a sectional view perpendicular to the base and parallel to the cross direction (CD),
- a dimension measured according to a view parallel to the base (51) and at a distance from the retainer elements (50, 51A),
- a cross dimension (CD) of the retainer elements (50, 50A), measured parallel to the upper face (511) of the base (51),
- a diametrical dimension of a portion of the retaining elements (50, 50A) in the case where the retaining elements (50, 50A) have, in at least some portions, a symmetry of revolution,
- the thickness of the rods of the retainer elements or the width of the heads (53) of the retaining elements (50, 50A), measured in a plane parallel to the upper face of the base (51),
**characterized in that**:
the retainer device has at least two retainer zones (RA, RB, RC) and an intermediate zone (RJ, RJ') disposed between the two retainer zones and connecting the two retainer zones, the retainer elements (50) disposed in the first and second retainer zones having values according to the first geometric dimension (h) which are greater than the values according to the first geometric dimension of the retainer elements (50A) disposed in the intermediate zone, wherein the assembly formed by the base (51) and the retainer elements (50, 50A) has a basis weight comprised between 10 and 120 g/m².

2. The retainer device according to claim 1, wherein the retainer zones (RA, RB, RC) and the intermediate zone (RJ, RJ') form elongated zones in the form of tapes in a common tape (R), the elongated zones and the common tape being optionally elongated along the longitudinal direction (MD).

3. The retainer device according to claim 1 or 2, wherein the intermediate zone (RJ, RJ') has at least one part in which the base (51) has a thickness smaller than a thickness (eb) of the base in the retainer zones (RA, RB, RC).

4. The retainer device according to any of claims 1 to 3, wherein, in the intermediate zone (RJ, RJ'), the base (51) has voids (50B).

5. The retainer device according to any of claims 1 to 4, wherein, in the retainer zones (RA, RB, RC), the retainer elements (51) have a value according to the first geometric dimension (h) which is substantially constant.

6. The retainer device according to any of claims 1 to 5, wherein, considered in the direction going from the first retainer zone (RA) to the second retainer zone (RB), the intermediate zone (RJ) has successively retainer elements (50A) whose value according to the first geometric dimension decreases then retainer elements whose value according to the first geometric dimension increases.

7. The retainer device according to any of claims 1 to 6, wherein any straight line passing through at least one retainer element (50, 50A) and extending along the direction going from the first retainer zone (RA) to the second retainer zone (RB) while passing through said first and second retainer zones, intersects at least 3 retainer elements particularly at least 5 retainer elements in each of the first and second retainer zones and, optionally, at least 1 retainer element, particularly at least 2 retainer elements in the intermediate zone.

8. The retainer device according to any of claims 1 to 7, wherein, at least in the retainer zones (RA, RB, RC), the retainer elements (50) are each formed of a rod (52) surmounted by a head (53) protruding from the rod, at least part of the retainer elements of the intermediate zone being optionally devoid of a head.

9. The retainer device according to any of claims 1 to 8, wherein the assembly formed by the base (51) and the retainer elements (50, 50A) has a basis weight comprised between 30 and 80 g/m², in some cases between 30 and 70 g/m², more particularly between 50 and 70 g/m².

10. The retainer device according to any of claims 1 to 9, further comprising a substrate (60) carrying the base, the substrate optionally comprising a layer of nonwoven material.

11. An absorbent item (200) of the baby diaper or adult incontinence diaper type, the item comprising an assembly which comprises two external sheets (210, 220) and an absorbent core (230) disposed between the external sheets, the assembly being arranged so as to present a first face of the diaper (FA), particularly a front face, and sides of the diaper (CC), the item comprising a hook-and-loop retainer system comprising receiving loops (240) carried by one of the elements among the first face of the diaper and one of the sides of the diaper, and at least one retainer device (5) according to any of claims 1 to 10, carried by the other of the elements among the first face of the diaper and said one of the sides of the diaper, so that the retainer elements cooperate with the receiving loops when said at least one of the sides of the diaper is placed against the first face of the diaper to retain said at least one of the sides of the diaper with respect to said first face.

12. A method for manufacturing a retainer device as defined in claim 1, wherein:
- a molding device (1) is provided having a plurality of cavities (13) formed in a recessed manner from a surface (12),
- a heated molding material (M) is applied on said surface, using an applicator (3) by allowing the molding material to penetrate into the cavities (13) to form retainer elements,
**characterized in that** are generated two separate adjacent flows (FA, FB and/or FC) of a molding material (M) applied on the surface (12) in two application zones by causing the molding material to flow so that the two flows meet in a junction zone so as to form a base (51), so that the material penetrates further into the cavities present in the application zones than into the cavities present in the junction zone.

13. The method according to claim 12, wherein the applicator (3) is an extrusion device comprising two adjacent channels (3A, 3B and/or 3C) separated by a partition (3', 3"), and the molding material (M) is applied by moving the molding device (1) and the extrusion device (3) relative to each other in a longitudinal direction.

14. The method according to any of claims 12 to 13, wherein, before cooling of the molding material (M), a substrate (60) is applied against the plastic material applied on the surface of the molding device (1), so that said material is sandwiched between the surface of the molding device and the substrate.
Retainer device comprising a base (51) extending along a longitudinal direction and a plurality of retainer elements (50, 50A) extending from the upper face of the base, each retainer element having a value according to a first geometric dimension. The retainer device has at least two retainer zones (RA, RB, RC) and an intermediate zone (RJ, RJ') disposed between the two retainer zones and connecting the two retainer zones, the retainer elements (50) disposed in the first and second retainer zones having values according to the first geometric dimension (h) which are greater than the values according to the first geometric dimension of the retainer elements (50A) disposed in the intermediate zone.
